# EUROPEAN PATENT APPLICATION

(11) **EP 3 366 213 A1**
(43) Date of publication of application: **29.08.2018**
(21) Application number: 15906527.5
(22) Date of filing: 24.10.2015
(51) Int. Cl.: A61B 5/11, A61B 5/0205

(54) **SLEEP EVALUATION DISPLAY METHOD AND APPARATUS, AND EVALUATION DEVICE**

(71) Applicant: Shenzhen Medica Technology Development Co., Ltd., Shenzhen, Guangdong 518057 (CN)
(72) Inventor: DAI, Peng, Shenzhen Guangdong518057 (CN); LI, Ming, Shenzhen Guangdong 518057 (CN); CAI, Jieling, Shenzhen Guangdong 518057 (CN); HUANG, Jianxin, Shenzhen Guangdong 518057 (CN); SHEN, Jinpeng, Shenzhen Guangdong 518057 (CN); CHEN, Zhenkun, Shenzhen Guangdong 518057 (CN); HUANG, Jinfeng, Shenzhen Guangdong 518057 (CN)
(74) Representative: Viering, Jentschura & Partner mbB Patent- und Rechtsanwälte
(86) International application number: PCT/CN2015/092783
(87) International publication number: WO 2017/067010

(57) **Abstract**

The present application relates to a sleep evaluation display method and device and an evaluation equipment. The method includes: acquiring a sleep monitoring signal; analyzing the sleep monitoring signal in sequence based on a plurality of scoring items according to a signal parsing strategy corresponding to each of the scoring items to obtain signal parsing results corresponding to a plurality of scoring items respectively; searching for a corresponding relationship between each of the scoring items and a corresponding step scoring coefficient according to the signal parsing results corresponding to the plurality of scoring items respectively, and determining the step scoring coefficients corresponding to each of the scoring items; summarizing the step scoring coefficients of all the scoring items to obtain an overall evaluation result of the sleep monitoring signal; and prompting the overall evaluation result and the step scoring coefficients related thereto. Requirements of high adaptability and high monitoring result reliability of a user on sleep monitoring are met.

## Description

### TECHNICAL FIELD

The present application relates to a sleep monitoring technology, and more particularly relates to a method and a device for sleep evaluation display and an evaluation equipment applicable to terminal equipment such as a mobile phone.

### BACKGROUND

The quality of sleep has crucial impact on human health. Monitoring some physiological parameters in sleep may not only evaluate the quality of sleep, but also provide a strong diagnostic basis for certain diseases. On such a basis, researching and implementing a noncontact sleep monitoring method is of great significance.

A monitoring process of an existing sleep monitoring method is as follows: a body movement signal is collected during the sleep of a monitored person, and one hard-decision is made every time when a signal is collected, so as to decide the current sleep level of the monitored person. Since the decision is made every time when a signal is collected, the collected signals are easily interfered, resulting in decision errors and poor reliability of statistical data. The distances between the sensor and the monitored person are different, which results in sensitivity deviation of the sensor and poor self-adaptability of adopting the hard-decision, so it is impossible to accurately monitor the sleep status of the monitored person. Therefore, the existing sleep monitoring method has poor anti-interference capability, poor adaptability and low reliability of monitoring result. Moreover, in existing sleep evaluation methods, usually only one kind of signal is collected to evaluate overall sleep quality on the basis of a certain criterion. Such an evaluation manner is relatively simple, and the flexibility and accuracy are not enough to satisfy the requirements of a user on more sleep monitoring details. Therefore, the existing sleep monitoring methods cannot meet the needs of the users for high self-adaptability and high reliability of monitoring result in sleep monitoring.

### SUMMARY

In order to solve the above-mentioned technical problems, the present application provides a sleep evaluation display method and device and an evaluation equipment applicable to a mobile terminal such as a mobile phone, so as to satisfy the requirements of a user for high adaptability and high reliability of monitoring results in sleep monitoring.

An embodiment of the present application provides a sleep evaluation display method including:
acquiring a sleep monitoring signal;
analyzing the sleep monitoring signal in sequence based on a plurality of scoring items according to a signal parsing strategy corresponding to each of the scoring items, so as to obtain signal parsing results corresponding to the plurality of scoring items respectively;
searching for a corresponding relationship between each of the scoring items and a corresponding step scoring coefficient according to the signal parsing results corresponding to the plurality of scoring items respectively, and determining a step scoring coefficient corresponding to each of the scoring items;
summarizing step scoring coefficients of all the scoring items to obtain an overall evaluation result of the sleep monitoring signal; and
prompting the overall evaluation result and a step scoring coefficient related thereto .

An embodiment of the present application further provides a sleep evaluation display device including:
a signal acquisition module configured to acquire a sleep monitoring signal;
a type-by-type analysis module configured to analyze the sleep monitoring signal in sequence based on a plurality of scoring items according to a signal parsing strategy corresponding to each of the scoring items, so as to obtain signal parsing results corresponding to the plurality of scoring items respectively;
a searching module configured to search for a corresponding relationship between each of the scoring items and a corresponding step scoring coefficient according to the signal parsing results corresponding to the plurality of scoring items respectively, and determine a step scoring coefficient corresponding to each of the scoring items;
a calculation module configured to summarize step scoring coefficients of all the scoring items to obtain an overall evaluation result of the sleep monitoring signal; and
a prompting module configured to prompt the overall evaluation result and a step scoring coefficient related thereto.

An embodiment of the present application further provides a sleep evaluation equipment including:
a storage module configured to store a signal parsing strategy corresponding to each of scoring items and a corresponding relationship between each of the scoring items and a corresponding step scoring coefficient;
an interface module configured to acquire a sleep monitoring signal;
a processor configured to analyze the sleep monitoring signal in sequence based on a plurality of scoring items according to the signal parsing strategy corresponding to each of the scoring items to obtain signal parsing results corresponding to the plurality of scoring items respectively, search for a corresponding relationship between each of the scoring items and a corresponding step scoring coefficient according to the signal parsing results corresponding to the plurality of scoring items respectively, determine a step scoring coefficient corresponding to each of the scoring items, and summarize step scoring coefficients of all the scoring items to obtain an overall evaluation result of the sleep monitoring signal; and
a display and/or an audio output module configured to display the overall evaluation result and a step scoring coefficient related therewith through the display, and/or broadcast the overall evaluation result and the step scoring coefficients related thereto through the audio output module.

### BRIEF DESCRIPTION OF THE DRAWINGS

Fig. 1 is a schematic diagram of a hardware structure in an embodiment of the present application;
Fig. 2 is a flow chart of a method in an embodiment of the present application;
Fig. 3 is a schematic diagram of an interface in an embodiment of the present application;
Fig. 4 is a schematic diagram of an interface in an embodiment of the present application;
Fig. 5 is a schematic diagram of an interface in an embodiment of the present application;
Fig. 6 is a schematic diagram of a device structure in an embodiment of the present application.

### DETAILED DESCRIPTION

For meeting requirements of high adaptability and high reliability of the monitoring result for a user on sleep monitoring, an embodiment of the present application provides a sleep evaluation equipment, which may be terminal equipment running a sleep evaluation display method, for example, a mobile phone, an IPAD and a computer, and is particularly applied to mobile terminal equipment. In the embodiment of the present application, overall sleep quality is evaluated by setting a plurality of scoring items, thereby ensuring a more accurate evaluation of sleep quality, providing the user a larger amount of information, and achieving a higher reliability of monitoring result.

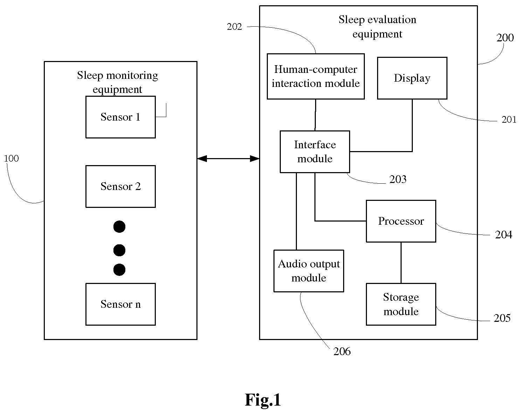

As shown in Fig. 1, a sleep evaluation equipment 200 acquires a sleep monitoring signal from a sleep monitoring equipment 100, and the sleep monitoring signal may at least includes one of: a body movement signal, a sleep heart rate signal, a sleep breathing rate signal, a breath noise signal, a wave signal and the like. Usually, at least one sensor is arranged on the sleep monitoring equipment 100, and the sensor may include at least one of or a combination of two or more of: a piezoelectric sensor (such as a piezoelectric thin film), a pressure sensor (such as hydraulic pressure, air pressure, etc.), a microphone, a capacitor, a radar, an acceleration sensor or/and a gyroscope, a photoelectric sensor, an electroencephalogram signal monitoring sensor, an electrooculogram signal monitoring sensor, an electromyogram signal monitoring sensor and the like, and the monitored signal is calculated and processed into a signal parameter in the electronic equipment. For example, using an acceleration sensor or/and a gyroscope to monitor vibration, the sleep monitoring equipment 100 may be a mobile phone, a bracelet, a sleep vibrator and the like. For another example, using piezoelectric or pressure to monitor a heart rate, a breathing rate and a body movement, the sleep monitoring equipment 100 may exist in a form of a non-wearing monitoring equipment. For another example, using a radar to detect turning over and a movement of the chest in breathing, the sleep monitoring equipment 100 may exist in a form of a radar radio transmitter. As such, the type of sensor placed-in is determined according to the type of sleep monitoring signals required to be monitored.

The sleep evaluation equipment 200 may acquire the sleep monitoring signal from the sleep monitoring equipment 100 through a serial port in the equipment, a wireless network, a data line and the like. The sleep monitoring equipment 100 may be arranged in the sleep evaluation equipment 200. For example, the sleep evaluation equipment 200 may be terminal equipment such as a mobile phone, an IPAD, a computer, etc., which has each of the abovementioned sensors and runs the sleep evaluation display method.

The sleep evaluation equipment 200 may include an interface module 203, a display 201, a processor 204 and a memory 205. The processor 204 is connected with the memory 205, and the display 201 is connected with the processor 204 through the interface module 203. The memory 205 is configured to store a strategy for acquiring data, analyzing the data, and the like. The memory 205 is a storage chip or a combination of storage chips, which includes equipment such as a hard disk, a cache, and the like. The display 201 is configured to display visual information on an interface, and it may be a touch display screen. The processor 204 is configured to process output and run an executive program stored in the memory, and may be one or more processors or controllers. The interface module 203 is a signal transmission channel between the processor 204 and a peripheral, including: a wired interface equipment and/or a wireless interface module, and a corresponding type of the interface module 203 is selected according to an access manner of a human-computer interaction module 202 and the display 201. The sleep evaluation equipment 200 may further include the human-computer interaction module 202. The human-computer interaction module 202 is connected to the processor 204 through the interface module 203 for interacting with a user and acquiring control instructions input by the user. The sleep evaluation equipment 200 may further include an audio output module 206, such as a loudspeaker, a sound and an earphone, and the like, and a voice prompt may be broadcasted through the audio output module. The audio output module 206 is connected to the processor 204 through the interface module 203.

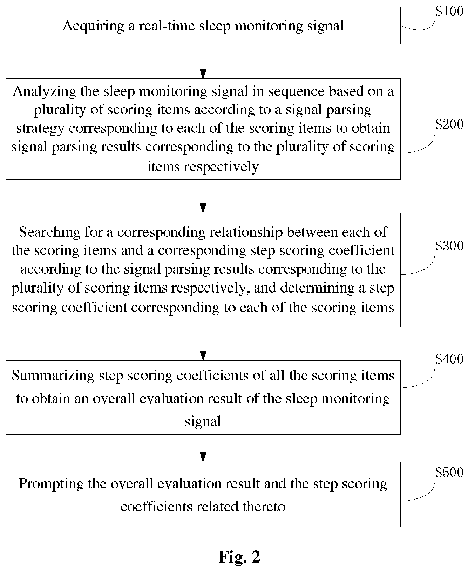

A flow chart shown in Fig. 2 provides a sleep evaluation display method in an embodiment of the present application.

In Step S100, a sleep evaluation equipment 200 acquires a sleep monitoring signal from a sleep monitoring equipment 100. In addition, the sleep evaluation equipment 200 may acquire the sleep monitoring signal from the sleep monitoring equipment 100 through an interface module 203. Here, the sleep monitoring signal may at least include: one of a body movement signal, a sleep heart rate signal, a sleep breathing rate signal, a breath noise signal, an electroencephalogram signal, an electrooculogram signal, an electromyogram signal, blood oxygen saturation detection data and the like. According to scoring items, the sleep monitoring signal may be at least one signal or a combination of two or more signals selected from the body movement signal, the sleep heart rate signal, the sleep breathing rate signal, the breath noise signal, the electroencephalogram signal, the electrooculogram signal, the electromyogram signal and the blood oxygen saturation detection data. The body movement signal may be acquired through a signal of a piezoelectric sensor. The breath noise signal may be acquired through an external sound receiving sensor. The sleep heart rate signal and the sleep breathing rate signal may be acquired through corresponding sensors, or the sleep heart rate signal and the sleep breathing rate signal are separated and acquired from the monitoring signals by using signal time domain analysis, frequency domain analysis, time-frequency analysis and the like. The electroencephalogram signal, the electrooculogram signal and the electromyogram signal may be acquired through a wave sampling sensor. A pulse rate (consistent with a heart rate under a normal condition) and a breathing rate are analyzed in a blood oxygen collection manner.

The sleep monitoring signal acquired in the embodiment may be a real-time monitoring signal, and may also be a non-real-time monitoring signal.

The sleep evaluation equipment 200 may acquire the sleep monitoring signal from the sleep monitoring equipment 100 through a wireless network or a wired connection.

In Step S200, a processor 204 analyzes the sleep monitoring signal in sequence based on a plurality of scoring items ("a plurality of' refers to two or more than two in this description) according to a signal parsing strategy corresponding to each of the scoring items, so as to obtain signal parsing results corresponding to a plurality of scoring items respectively.

In the embodiment, the scoring items mentioned-above at least include two scoring items in the following types:
1. A plurality of scoring items related to the heart rate, for example, a bradycardia, a heart rate, an arrhythmia (referring to alternately fast and slow heartbeats exceeding a general range), a tachycardia and a heartbeat pause frequency;
2. A plurality of scoring items related to the breathing rate, for example, a breathing rate, a tachypnea, a bradypnea and an apnea frequency;
3. A plurality of scoring items related to sleep time, for example, a duration for falling asleep, an actual sleep time point, a deep sleep proportion, a sleep duration, a Rapid-Eye-Movement (REM) sleep proportion (REM proportion), an Non-Rapid-Eye-Movement (NREM) sleep proportion (NREM proportion), an NREM sleep stage-1 duration, an NREM sleep stage-2 duration, an NREM sleep stage-3 duration and an NREM sleep stage-4 duration, etc., where, in a period of time during sleep, the frequency of brain waves becomes faster and the amplitude of brain waves becomes lower. At the same time, it also shows an increase both in heart rate and blood pressure, as well as the muscle relaxation and penile erection. Most strangely, eyeballs keep moving left and right. The sleep in such a stage is called REM sleep or paradoxical sleep, and someone also calls it active sleep. The other sleep except REM sleep is called slow wave sleep or quiet sleep, i.e., NREM sleep, where the slow wave sleep is divided into stage-1 (shallow sleep stage), stage-2 (light sleep stage), stage-3 (moderate sleep stage) and stage-4 (deep sleep stage), and then the REM sleep proportion refers to a proportion of the REM sleep duration to the sleep duration, and the NREM sleep proportion (the NREM proportion) refers to a sleep duration proportion of the stage-1 (shallow sleep stage), stage-2 (light sleep stage), stage-3 (moderate sleep stage) and stage-4 (deep sleep stage);
4. A plurality of scoring items related to body movement information, for example, a waking frequency, a waking time, an off-bed frequency, off-bed time and a body movement and turning-over frequency, etc.; and
5. A scoring item related to blood oxygen saturation.

Thus, it can be seen that the above-mentioned scoring items at least include at least two of the plurality of scoring items related to the heart rate, the plurality of scoring items related to the breathing rate, the plurality of scoring items related to the sleep time, the plurality of scoring items related to the body movement information and the scoring item related to the blood oxygen saturation.

For example, regarding the plurality of scoring items related to the heart rate, such as the bradycardia, the heart rate, the arrhythmia, the tachycardia and the heartbeat pause frequency, in Step S200, in an embodiment of the present application, signal parsing results corresponding to the plurality of scoring items related to the sleep heart rate are obtained according to the sleep heart rate signal. For example, in an embodiment of the present application, an average frequency of the sleep heart rate signal is determined as the signal parsing result corresponding to the scoring item related to the heart rate. In an embodiment of the present application, the signal parsing result corresponding to the scoring item related to bradycardia is obtained according to a judgment result of whether the average frequency of the heart rate signal is less than a preset threshold or not. In an embodiment of the present application, the signal parsing result corresponding to the scoring item related to tachycardia is obtained according to a judgment result of whether the average frequency of the heart rate signal is more than the preset threshold or not. In an embodiment of the present application, the signal parsing result corresponding to the scoring item of arrhythmia is obtained according to a frequency range of the heart rate signal. In an embodiment of the present application, the heartbeat pause frequency is obtained as the signal parsing result corresponding to the corresponding scoring item according to an abnormity frequency which means there is a breathing signal but no heartbeat signal.

For another example, regarding the plurality of scoring items related to the breathing rate, such as the breathing rate, the tachypnea, the bradypnea and the apnea frequency,
in Step S200, in an embodiment of the present application, signal parsing results corresponding to the plurality of scoring items related to the sleep breathing rate are obtained according to the sleep breathing rate signal. For example, in an embodiment of the present application, an average frequency of the sleep breathing rate signal is determined as the signal parsing result corresponding to the scoring item related to the breathing rate. In an embodiment of the present application, the signal parsing result corresponding to the scoring item of bradypnea is obtained according to a judgment result of whether the average frequency of the sleep breathing rate signal is less than a preset threshold or not. In an embodiment of the present application, the signal parsing result corresponding to the scoring item of tachypnea is obtained according to a judgment result of whether the average frequency of the sleep breathing rate signal is more than the preset threshold or not. In an embodiment of the present application, the apnea frequency is obtained as the signal parsing result corresponding to the corresponding scoring item according to an abnormity frequency which means there is a heartbeat signal but no breathing signal.

For another example, regarding the plurality of scoring items related to the sleep time, such as the duration for falling asleep, the actual sleep time point, the deep sleep proportion, the sleep duration, the REM sleep proportion (REM proportion), the NREM sleep proportion (NREM proportion), the NREM sleep stage-1 duration, the NREM sleep stage-2 duration, the NREM sleep stage-3 duration and the NREM sleep stage-4 duration,
in an embodiment of the present application, in Step S200, integral analysis is performed on a combination of at least one wave signal of the electroencephalogram signal, the electrooculogram signal and the electromyogram signal, and at least one signal of the body movement signal, the sleep heart rate signal and the sleep breathing rate signal, to obtain signal parsing results corresponding to the plurality of scoring items related to the sleep time. Detailed descriptions will be made below. For example, in Step S200, in an embodiment of the present application, starting time and ending time of the sleep monitoring signal are analyzed to obtain the signal parsing result corresponding to the sleep duration. In Step S200, in an embodiment of the present application, a heartbeat signal and/or a breathing signal are/is separated from the sleep monitoring signal, and then the heart rate and/or the breathing rate are/is further extracted. When the frequencies/a frequency of the heart rate and/or the breathing rate signals/signal are/is lower than a threshold value, a time point is recorded. The signal parsing result corresponding to the duration for falling asleep is obtained through the starting time of the sleep monitoring signal and the time point. The time point is determined as the actual sleep time point. Of course, a plurality of manners may further be adopted for a falling-asleep judgment method. For example, a sleep state is obtained by determining that the heart rate, the breathing rate and/or the body movement frequency are/is less than a threshold or by analyzing waves/a wave of the electroencephalogram signal, the electrooculogram signal and/or the electromyogram signal, and the actual sleep time point is further obtained.

In Step S200, in an embodiment of the present application, the frequencies/frequency of the heart rate and/or breathing rate signals/signal and an occurrence frequency of the body movement signal are analyzed, the sleep depths are divided, and the deep sleep duration and light sleep duration are calculated, thus, the signal parsing result corresponding to the deep sleep proportion is obtained. The sleep duration is calculated and obtained according to the actual sleep time point and the time when no sleep monitoring signal is acquired for a long time, as the signal parsing result corresponding to the corresponding scoring item. Of course, the deep sleep proportion may further be analyzed in conjunction with an analytical processing result of at least one wave signal of the electroencephalogram signal, the electrooculogram signal and the electromyogram signal, and such an analysis manner is more accurate.

In Step S200, in an embodiment of the present application, signal parsing results/a signal parsing result corresponding to the REM sleep proportion and/or the NREM sleep proportion, the NREM sleep stage-1 duration, the NREM sleep stage-2 duration, the NREM sleep stage-3 duration and the NREM sleep stage-4 duration are obtained according to at least one wave signal of the electroencephalogram signal, the electrooculogram signal and the electromyogram signal.

For another example, regarding the plurality of scoring items related to the body movement information, for example, the waking frequency, the waking time, the off-bed frequency, the off-bed time and the body movement and turning-over frequency,
in Step S200, in an embodiment of the present application, the body movement signal is extracted from a disturbance signal existing in the sleep monitoring signal, and a frequency of the body movement signal is analyzed to obtain a signal parsing result corresponding to the body movement and turning-over frequency.

In Step S200, in an embodiment of the present application, a frequency of no signal input in the body movement signal, or a frequency of no signal input in the sleep heart rate signal and the breathing rate signal is parsed to obtain a signal parsing result corresponding to the off-bed frequency. Alternatively, a pressure signal may also be obtained through the piezoelectric sensor in the sleep time, and a frequency of no signal input in the pressure signal is analyzed and used as the off-bed frequency.

In Step S200, in an embodiment of the present application, a signal parsing result corresponding to the scoring item of the waking frequency is determined according to whether the frequencies/frequency of the heart rate signal and/or the breathing rate signal are/is higher than a threshold value or not, and of course, the waking frequency may further include: the body movement and turning-over frequency, the off-bed frequency and the like.

For another example, regarding the scoring item of the blood oxygen saturation, a measurement method for the blood oxygen saturation includes a fingertip photoelectric sensor. During measurement, it is only necessary to sleeve the user's finger with the sensor, the finger is utilized as a transparent container which contains hemoglobin, the red light and near infrared light with wavelengths of 660nm and 940nm respectively are used as incident light sources, and a conduction intensity of light passing through a tissue bed is determined to calculate a hemoglobin concentration and the blood oxygen saturation.

The signal parsing strategies corresponding to the plurality of scoring items provided in the abovementioned embodiments are adopted to obtain the signal parsing results corresponding to the scoring items. Of course, each of the abovementioned scoring items is also not limited to the abovementioned signal parsing strategies, and corresponding analysis results may further be obtained by adopting other signal analysis strategies, which will not be listed herein.

The signal parsing strategies corresponding to the scoring items are selected and determined one by one according to the number of the scoring items. For example, if there is only one scoring item included, it is only necessary to adopt one corresponding signal parsing strategy, and if there are a plurality of scoring items included, it is necessary to adopt a plurality of corresponding signal parsing strategies. In Step S300, the processor 204 searches for a corresponding relationship between each of the scoring items and a corresponding step scoring coefficient according to the signal parsing results corresponding to the plurality of scoring items respectively, and determines a step scoring coefficient corresponding to each of the scoring items.

In the embodiment, a corresponding relationship between each of the scoring items and a corresponding step scoring coefficient is pre-stored in a memory to construct data corresponding relationship, for example, in a table form. Each of the scoring items is divided into different stages according to reference ranges, and each stage corresponds to a step scoring coefficient, specifically referring to Table 1 shown as follows. A step scoring coefficient may be a score, a proportion, or a formula. The formula may determine a corresponding coefficient through more other coefficients and calculation methods.

**Table 1**

| Scoring item | Stage | | Step scoring coefficient |
|---|---|---|---|
| Actual sleep time point | z1-z2 | | a1 |
| | z2-z3 | | a2 |
| | ... | | |
| Duration for falling asleep | z1-z2 | | b1 |
| | z2-z3 | | b2 |
| | ... | | |
| Off-bed (getting up in the night to urinate) frequency | z1-z2 | | c1 |
| | z2-z3 | | c2 |
| | ... | | |
| Waking frequency | z1-z2 | | d1 |
| | z2-z3 | | d2 |
| | ... | | |
| Deep sleep proportion | z1-z2 | | e1 |
| | z2-z3 | | e2 |
| | ... | | |
| Body movement and turning-over frequency (restless) | z1-z2 | | f1 |
| | z2-z3 | | f2 |
| | ... | | |
| Apnea frequency | 0 | | g1 |
| | More than 0 | | g2 |
| Breathing rate | z1-z2 (breathing rate) | t1-t2 (time) | h1 |
| | | t2-t3 (time) | h2 |
| | | ... | |
| | z2-z3 (breathing rate) | t1-t2 (time) | h3 |
| | | t2-t3 (time) | h4 |
| | | ... | |
| | ... | | |
| Heartbeat pause frequency | 0 | | i1 |
| | More than 0 | | i2 |
| Heart rate | z1-z2 (heart rate) | t1-t2 (time) | j1 |
| | | t2-t3 (time) | j2 |
| | | ... | |
| | z2-z3 (heart rate) | t1-t2 (time) | j3 |
| | | t2-t3 (time) | j4 |
| | | ... | |
| | z3-z4 (heart rate) | t1-t2 (time) | j5 |
| | | t2-t3 (time) | j6 |
| | | ... | |
| | ... | | |
| Arrhythmia | The heart rate is within the ranges of z1-z2 and z2-z3 | | j1 +j3+... |
| | The heart rate is within the ranges of z2-z3 and z3-z4 | | j3+j5+... |
| Sleep duration | z1-z2 | | m1 |
| | z2-z3 | | m2 |
| | ... | | |
| Blood oxygen saturation | z1-z2 | | n1 |
| | z2-z3 | | n2 |
| | ... | | |
| REM sleep proportion | z1-z2 | | o1 |
| | z2-z3 | | o2 |
| | ... | | |
| NREM sleep proportion | z1-z2 | | p1 |
| | z2-z3 | | p2 |
| | ... | | |

From the scoring items listed in the above table, it can be seen that each of the scoring items may be divided into different stages according to the reference ranges, and here, the reference ranges may be time reference ranges and/or numerical value reference ranges. For example, for the heart rate or the breathing rate, in addition to performing a division of a first stage according to the average frequency ranges of the heart rate or the breathing rate, a division of a second stage is also performed on each of the average frequency range of the first stage according to the duration, so that the corresponding step scoring coefficients are correspondingly set for each average frequency range and duration. In addition, for arrhythmia, its step scoring coefficient may be a combination of the step scoring coefficients corresponding to the scoring items of the heart rate. For example, when the heart rate is within both the ranges of z2-z3 and z3-z4, the step scoring coefficient corresponding to arrhythmia is equal to a sum of the step scoring coefficient corresponding to the heart rate within the range of z2-z3 and the step scoring coefficient of the heart rate within the range of z3-z4. Of course, only part of scoring items are listed in the table, and scoring items which are not listed may be set with reference to contents of the table.

In the embodiment, according to the reference ranges, each of the scoring items is divided into different stages, and the number of stages may be one or more than one. For example, in the above table, most of the scoring items correspond to at least two stages. Of course, for some scoring items, only one stage may also be adopted. For example, for the heartbeat pause frequency and the apnea frequency, it is possible to adopt only the stages corresponding to the frequency of 0.

In Step S400, the processor 204 summarizes step scoring coefficients of all the scoring items to obtain an overall evaluation result of the sleep monitoring signal.

In an embodiment of the present application, the step scoring coefficient corresponding to each of the scoring items is a numerical value or a percentage, and a manner for summarizing the step scoring coefficients of all the scoring items may adopt one of the following manners.

A first manner: the step scoring coefficients corresponding to all the scoring items are summarized to obtain the overall evaluation result of the sleep monitoring signal. For example, the overall evaluation result of the sleep monitoring signal is equal to a2+b1+c2+d2+e1+f1+.......

A second manner: the step scoring coefficients corresponding to all the scoring items are subtracted from a basic score to obtain the overall evaluation result of the sleep monitoring signal. For example, the overall evaluation result of the sleep monitoring signal is equal to 100-a2-b1-c2-d2-e1-f1- .......

A third manner: weighted averaging is performed on the step scoring coefficients corresponding to all the scoring items to obtain the overall evaluation result of the sleep monitoring signal. For example, evaluation is performed for each scoring item by taking 100 as a base number to obtain the corresponding step scoring coefficient, and then weighted averaging is performed on the step scoring coefficients corresponding to all the scoring items to obtain an average value as the overall evaluation result. Here, weighting coefficients for weighted averaging are set according to the influence of the scoring items on sleep quality.

The above-mentioned summarization or subtraction manner further includes that, the weighting coefficients are set for each of the scoring items according to the influence of the scoring items on the sleep quality. In the first or second manner, when performing the summarization or subtraction, each step scoring coefficient is multiplied by the corresponding weighting coefficient. For example, allocation of the weighting coefficients is shown in Table 2 below.

**Table 2**

| Scoring item | Weighting coefficient | Stage | | Step scoring coefficient |
|---|---|---|---|---|
| Actual sleep time point | K1 | z1-z2 | | a1 |
| | | z2-z3 | | a2 |
| | | ... | | |
| Duration for falling asleep | K2 | z1-z2 | | b1 |
| | | z2-z3 | | b2 |
| | | ... | | |
| Off-bed (getting up in the night to urinate) frequency | K3 | z1-z2 | | c1 |
| | | z2-z3 | | c2 |
| | | ... | | |
| Waking frequency | K4 | z1-z2 | | d1 |
| | | z2-z3 | | d2 |
| | | ... | | |
| Deep sleep proportion | K5 | z1-z2 | | e1 |
| | | z2-z3 | | e2 |
| | | ... | | |
| Body movement and turning-over frequency (restless) | K6 | z1-z2 | | f1 |
| | | z2-z3 | | f2 |
| | | ... | | |
| Apnea frequency | K7 | 0 | | g1 |
| | | More than 0 | | g2 |
| Breathing rate | K8 | z1-z2 (breathing rate) | t1-t2 (time) | h1 |
| | | | T2-t3 (time) | h2 |
| | | | ... | |
| | | z2-z3 (breathing rate) | t1-t2 (time) | h3 |
| | | | T2-t3 (time) | h4 |
| | | | ... | |
| | | ... | | |
| Heartbeat pause frequency | K9 | 0 | | i1 |
| | | More than 0 | | i2 |
| Heart rate | K13 | z1-z2 (heart rate) | t1-t2 (time) | j1 |
| | | | t2-t3 (time) | j2 |
| | | | ... | |
| | K14 | z2-z3 (heart rate) | t1-t2 (time) | j3 |
| | | | t2-t3 (time) | j4 |
| | | | ... | |
| | K15 | z3-z4 (heart rate) | t1-t2 (time) | j5 |
| | | | t2-t3 (time) | j6 |
| | | | t1-t2 (time) | |
| | | ... | | |
| Arrhythmia | K11 | The heart rate is within the ranges of z1-z2 and z2-z3 | | j1+j3+... |
| | | The heart rate is within the ranges of z2-z3 and z3-z4 | | j3+j5+... |
| Sleep duration | K12 | z1-z2 | | m1 |
| | | z2-z3 | | m2 |
| | | ... | | |
| Blood oxygen saturation | K13 | z1-z2 | | n1 |
| | | z2-z3 | | n2 |
| | | ... | | |
| REM sleep proportion | K14 | z1-z2 | | o1 |
| | | z2-z3 | | o2 |
| | | ... | | |
| NREM sleep proportion | K15 | z1-z2 | | p1 |
| | | z2-z3 | | p2 |
| | | ... | | |

When the first manner is adopted, the overall evaluation result of the sleep monitoring signal may be represented as K1*a2+K2*b1+K3*c2+K4*d2+K5*e1+K6*f1+.......

When the second manner is adopted, the overall evaluation result of the sleep monitoring signal may be represented as 100-K1*a2-K2*b1-K3*c2-K4*d2-K5*e1-K6*f1+.......

Of course, a weighting coefficient may further be allocated to each stage according to stage division of each of scoring items. Referring to the scoring item of the heart rate in the above table, where three weighting coefficients are set according to three stages corresponding to the scoring items of the heart rate respectively. Similarly, the other scoring items may also be set with reference to the scoring item of the heart rate. For each of the scoring items, a weighting coefficient is set for each stage according to stage divisions, that is, different weighting coefficients are set for a plurality of step scoring coefficients corresponding to the scoring items respectively, thereby achieving higher flexibility, facilitating the user to adjust a weighting ratio according to their own situation, and improving the user experience.

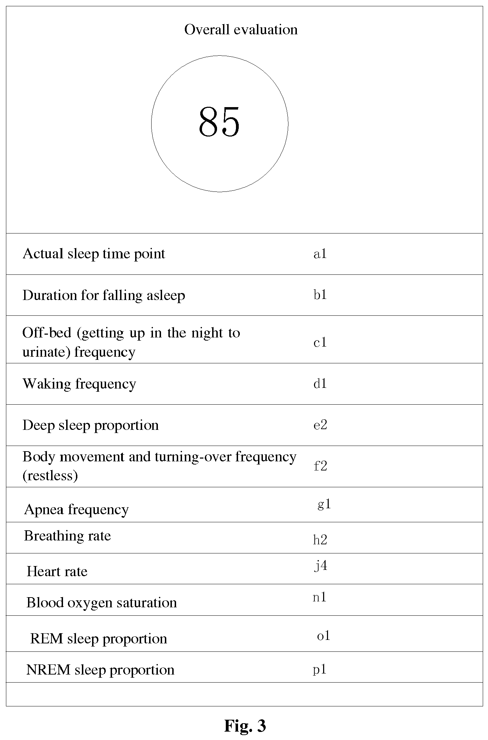

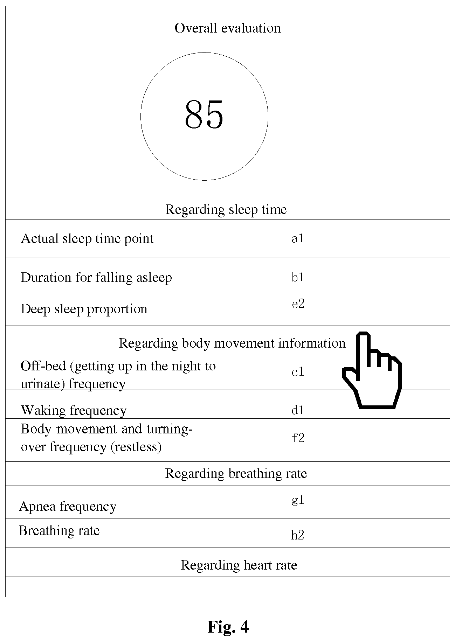

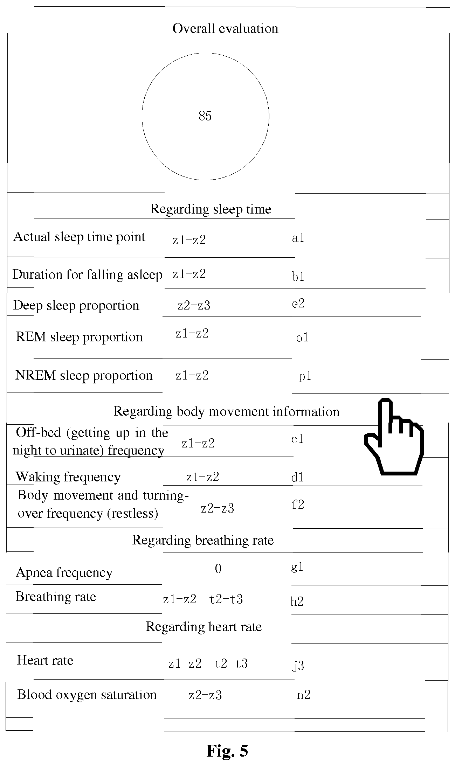

In Step S500, the overall evaluation result and the step scoring coefficients related thereto are displayed through a display 201, and/or the overall evaluation result and the step scoring coefficients related thereto are broadcasted through an audio output module 206. The display through the display 201 may adopt one of the following manners.
1. Referring to Fig. 3, the overall evaluation result and the step scoring coefficients corresponding to each of the scoring items for summarization are displayed.
2. Referring to Fig. 3, the overall evaluation result and the step scoring coefficients corresponding to all the scoring items are displayed. Alternatively, as shown in Fig. 4, the classification information is classified and displayed, and when the user clicks a classification item on a touch display screen through a finger 300, all scoring items and their corresponding step scoring coefficients under this classification item are extended. Here, the classification item includes the heart rate, the breathing rate, the body movement information, the sleep time and the like, and its scoring items related thereto refer to related descriptions made before.
3. Referring to Fig. 5, the overall evaluation result, the corresponding relationship between each of the scoring items for summarization and the corresponding step scoring coefficient, and the corresponding step scoring coefficients are displayed.
4. Referring to Fig. 5 and Fig. 4, the overall evaluation result, the corresponding relationships between all the scoring items and the corresponding step scoring coefficients, and the corresponding step scoring coefficients are displayed.

Of course, if the weighting coefficients corresponding to each of the scoring items are set, the corresponding weighting coefficients may also be displayed on a display interface.

Similarly, the broadcasting of the overall evaluation result and the step scoring coefficients related thereto through the audio output module may also adopt one of the following manners:
the overall evaluation result and the step scoring coefficient corresponding to each of the scoring items for summarization are broadcast;
the overall evaluation result and the step scoring coefficients corresponding to all the scoring items are broadcast;
the overall evaluation result, the corresponding relationship between each of the scoring items for summarization and the corresponding step scoring coefficients, and the corresponding step scoring coefficients are broadcast; and
the overall evaluation result, the corresponding relationships between all the scoring items and the corresponding step scoring coefficients, and the corresponding step scoring coefficients are broadcast.

In an embodiment of the present application, the sleep evaluation equipment 200 further includes:
a human-computer interaction module 202 configured to receive a selection instruction input by the user according to the number of the scoring items, or modify the weighting coefficients corresponding to each of the scoring items according to the selection instruction input by the user. Of course, for facilitating more intuitive information display and edition, it is necessary to display the scoring items, the weighting coefficients corresponding to the scoring items and/or the corresponding relationships between the scoring items and their corresponding step scoring coefficients on the display interface by virtue of the display, then the scoring items, the weighting coefficients and/or the corresponding relationships between the scoring items and their corresponding step scoring coefficients may be autonomously edited according to an instruction input by the user, and the edited results are saved for the next sleep evaluation.

For each flowchart in the drawings, it should be understood that each step in the drawings is sequentially displayed according to the indication of arrows, but these steps are not always sequentially executed according to a sequence indicated by the arrows. Unless expressly stated in the description, these steps are executed without any strict sequence limit, and they may be executed in another sequence. Moreover, at least part of steps in the drawings may include a plurality of sub-steps or a plurality of stages, these sub-steps or stages are not always executed and completed at the same moment, and may be executed at different moments, and they are not always executed sequentially.

From the above descriptions about the implementation modes, those skilled in the art may clearly know that the method of each embodiment may be implemented in a manner of combining software and a necessary universal hardware platform, and of course, may also be implemented through hardware, but the former is a preferred implementation mode under many circumstances. On the basis of such an understanding, the technical solutions of the present application substantially or parts making contributions to the prior art may be embodied in form of software product, and the computer software product is born in a nonvolatile computer-readable storage carrier (for example, a Read-Only Memory (ROM), a magnetic disk, a cloud disk and a server cloud space), including a plurality of instructions configured to enable a terminal equipment (which may be a mobile phone, a computer, a server, network equipment or the like) to execute the system structure and method of each embodiment of the present application.

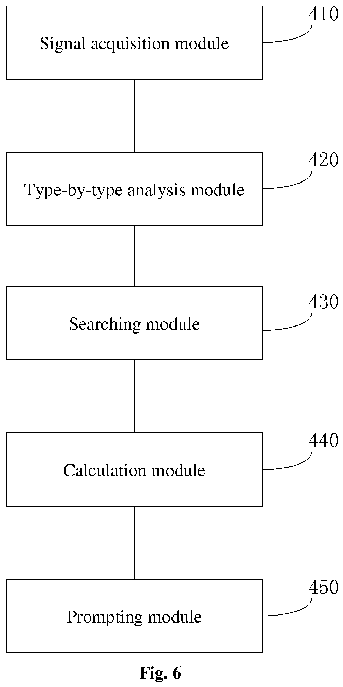

Fig. 6 shows a schematic diagram of a system architecture implementing the sleep evaluation display method. In an embodiment of the present application shown in Fig. 6, a sleep evaluation display device is provided, which includes:
a signal acquisition module 410 configured to acquire a sleep monitoring signal;
a type-by-type analysis module 420 configured to analyze the sleep monitoring signal in sequence based on the plurality of scoring items according to a signal parsing strategy corresponding to each of the scoring items, so as to obtain signal parsing results corresponding to a plurality of scoring items respectively;
a searching module 430 configured to search for a corresponding relationship between each of the scoring items and corresponding step scoring coefficients according to the signal parsing results corresponding to the plurality of scoring items respectively, and determine a step scoring coefficient corresponding to each of the scoring items;
a calculation module 440 configured to summarize step scoring coefficients of all the scoring items to obtain an overall evaluation result of the sleep monitoring signal; and
a prompting module 450 configured to prompt the overall evaluation result and the step scoring coefficients related thereto.

The abovementioned scoring items include at least two of: the bradycardia, the tachycardia, the actual sleep time point, the deep sleep proportion, the waking frequency, the waking time, the off-bed time, the tachypnea, the bradypnea, the apnea frequency, the off-bed frequency, the duration for falling asleep, the a body movement and turning-over frequency, the breathing rate, the a sleep duration, the heart rate, the arrhythmia, the heartbeat pause frequency, the REM sleep proportion, the NREM sleep proportion and the blood oxygen saturation.

The signal acquisition module 410, the type-by-type analysis module 420, the searching module 430, the calculation module 440 and the prompting module 450 are configured to respectively implement Step S100 to Step S500 in the abovementioned method, and thus a specific implementation of each module may refer to detailed descriptions of the related steps in the previous description, and will not be elaborated herein.

In an embodiment of the present application, a corresponding relationship between each of the scoring items and the corresponding step scoring coefficient is pre-stored to construct data corresponding relationships, for example, in a table form, wherein each of the scoring items is divided into different stages according to reference ranges, and each stage corresponds to a step scoring coefficient.

In an embodiment of the present application, the step scoring coefficient corresponding to each of the scoring items is a numerical value or a percentage; and
the calculation module 440 is configured to summarize the step scoring coefficients corresponding to all the scoring items to obtain the overall evaluation result of the sleep monitoring signal, or,
subtract the step scoring coefficients corresponding to all the scoring items from a basic score to obtain the overall evaluation result of the sleep monitoring signal, or,
perform weighted averaging on the step scoring coefficients corresponding to all the scoring items to obtain the overall evaluation result of the sleep monitoring signal.

In an embodiment of the present application, the prompting module at least includes one of the following units:
a first prompting unit configured to display or broadcast the overall evaluation result and the step scoring coefficient corresponding to each of the scoring items for summarization;
a second prompting unit configured to display or broadcast the overall evaluation result and the step scoring coefficients corresponding to all the scoring items;
a third prompting unit configured to display or broadcast the overall evaluation result, the corresponding relationship between each of the scoring items for summarization and the corresponding step scoring coefficients, and the corresponding step scoring coefficients; and
a fourth prompting unit configured to display or broadcast the overall evaluation result, the corresponding relationships between all the scoring items and the corresponding step scoring coefficients, and the corresponding step scoring coefficients.

In an embodiment of the present application, according to the scoring items, the sleep monitoring signal comprises at least one signal or a combination of two or more signals selected from a body movement signal, a sleep heart rate signal, a sleep breathing rate signal, a breath noise signal, an electroencephalogram signal, an electrooculogram signal, an electromyogram signal and blood oxygen saturation detection data.

In an embodiment of the present application, the type-by-type analysis module at least includes one of the following units:
a body movement analysis unit configured to obtain signal parsing results corresponding to a plurality of scoring items related to body movement information according to a body movement signal, or according to a body movement signal in conjunction with a sleep heart rate signal and/or a sleep breathing rate signal;
a heart rate analysis unit configured to obtain signal parsing results corresponding to a plurality of scoring items related to a sleep heart rate according to the sleep heart rate signal;
a breathing rate analysis unit configured to obtain signal parsing results corresponding to a plurality of scoring items related to a sleep breathing rate according to the sleep breathing rate signal; and
a sleep state analysis unit configured to obtain signal parsing results corresponding to a REM sleep proportion and/or an NREM sleep proportion according to at least one wave signal of the electroencephalogram signal, the electrooculogram signal and the electromyogram signal, and/or perform integral analysis on a combination of at least one wave signal of the electroencephalogram signal, the electrooculogram signal and the electromyogram signal and at least one of the body movement signal, the sleep heart rate signal and the sleep breathing rate signal, to obtain signal parsing results corresponding to a plurality of scoring items related to sleep time .

In an embodiment of the present application, the equipment further includes: a human-computer interaction module configured to acquire a selection instruction of a user, and edit the displayed scoring items, the weighting coefficients corresponding to the scoring items, and/or corresponding relationships between the scoring items and the corresponding step scoring coefficients according to the selection instruction.

In various embodiments of the present application, the scoring items are numerous, the classification is more meticulous, a scoring manner is more perfect, and a scoring result is more accurate, thereby satisfying the requirements of the user, and improving a user experience; and the settings related to the scoring items may be customized and modified according to the requirement of the user, thereby achieving higher flexibility in use.

Each technical characteristic of the abovementioned embodiments may be freely combined, and for brief description, not all possible combinations of each technical characteristic in the embodiments are described. However, any conflict-free combination of these technical characteristics shall fall within the scope recorded in the description.

The abovementioned embodiments only express some implementation modes of the present application, and they are described relatively more specifically and in more detail, but cannot be understood as limits to the scope of the present application. It should be pointed out that those skilled in the art may further make a plurality of variants and improvements without departing from the concept of the present application and all of them fall within the scope of protection of the present application. Therefore, the scope of protection of the present application shall be subject to the appended claims.

## Claims

1. A sleep evaluation display method, comprising:
acquiring a sleep monitoring signal;
analyzing the sleep monitoring signal in sequence based on a plurality of scoring items according to a signal parsing strategy corresponding to each of the scoring items, to obtain signal parsing results corresponding to the plurality of scoring items respectively;
searching for a corresponding relationship between each of the scoring items and a corresponding step scoring coefficient according to the signal parsing results corresponding to the plurality of scoring items respectively, and determining a step scoring coefficient corresponding to each of the scoring item;
summarizing step scoring coefficients of all the scoring items to obtain an overall evaluation result of the sleep monitoring signal; and
prompting the overall evaluation result and a step scoring coefficient related thereto.

2. The method according to claim 1, wherein the scoring items comprise at least two of: bradycardia, tachycardia, an actual sleep time point, a deep sleep proportion, a waking frequency, waking time, off-bed time, tachypnea, bradypnea, an apnea frequency, an off-bed frequency, a duration for falling asleep, a body movement and turning-over frequency, a breathing rate, a sleep duration, a heart rate, arrhythmia, a heartbeat pause frequency, a rapid-eye-movement (REM) sleep proportion, a non-rapid-eye-movement (NREM) sleep proportion and a blood oxygen saturation.

3. The method according to claim 1, wherein the corresponding relationship between each of the scoring items and the corresponding step scoring coefficient is pre-stored to construct data corresponding relationships, wherein each of the scoring items is divided into different stages according to reference ranges, and each stage corresponds to a step scoring coefficient.

4. The method according to claim 1, wherein the step scoring coefficient corresponding to each of the scoring items is a numerical value or a percentage; and
the summarizing the step scoring coefficients of all the scoring items to obtain the overall evaluation result of the sleep monitoring signal comprises:
summarizing the step scoring coefficients corresponding to all the scoring items to obtain the overall evaluation result of the sleep monitoring signal, or,
subtracting the step scoring coefficients corresponding to all the scoring items from a basic score to obtain the overall evaluation result of the sleep monitoring signal, or,
performing weighted averaging on the step scoring coefficients corresponding to all the scoring items to obtain the overall evaluation result of the sleep monitoring signal.

5. The method according to claim 4, wherein different weighting coefficients are set for the plurality of step scoring coefficients corresponding to the scoring items respectively to perform a calculation of summarization, subtraction or weighted averaging.

6. The method according to claim 1, wherein the prompting the overall evaluation result and the step scoring coefficients related thereto comprises:
displaying or broadcasting the overall evaluation result and the step scoring coefficient corresponding to each of the scoring items for summarization;
displaying or broadcasting the overall evaluation result and the step scoring coefficients corresponding to all the scoring items;
displaying or broadcasting the overall evaluation result, the corresponding relationship between each of the scoring items for summarization and the corresponding step scoring coefficient, and a corresponding step scoring coefficient; or,
displaying or broadcasting the overall evaluation result, the corresponding relationships between all the scoring items and the corresponding step scoring coefficients, and the corresponding step scoring coefficients.

7. The method according to claim 1, wherein according to the scoring items, the sleep monitoring signal comprises at least one signal or a combination of two or more signals selected from a body movement signal, a sleep heart rate signal, a sleep breathing rate signal, a breath noise signal, an electroencephalogram signal, an electrooculogram signal, an electromyogram signal and blood oxygen saturation detection data.

8. The method according to claim 1, wherein the analyzing the sleep monitoring signal in sequence based on a plurality of scoring items according to a signal parsing strategy corresponding to each of the scoring items, to obtain signal parsing results corresponding to the plurality of scoring items respectively comprises at least one of the following steps:
obtaining signal parsing results corresponding to a plurality of scoring items related to a sleep breathing rate according to the sleep breathing rate signal;
obtaining signal parsing results corresponding to a plurality of scoring items related to a sleep heart rate according to the sleep heart rate signal;
obtaining signal parsing results corresponding to a plurality of scoring items related to body movement information according to the body movement signal, or according to the body movement signal in conjunction with the sleep heart rate signal and/or the sleep breathing rate signal;
obtaining a signal parsing result corresponding to a REM sleep proportion and/or an NREM sleep proportion according to at least one of the electroencephalogram signal, the electrooculogram signal and the electromyogram signal; and
performing integral analysis on a combination of at least one of the electroencephalogram signal, the electrooculogram signal and the electromyogram signal, and at least one signal of the body movement signal, the sleep heart rate signal and the sleep breathing rate signal, to obtain signal parsing results corresponding to a plurality of scoring items related to sleep time.

9. The method according to claim 1 or 4, wherein the method further comprises:
displaying the scoring items, weighting coefficients corresponding to the scoring items, and/or the corresponding relationships between the scoring items and the corresponding step scoring coefficients; and
acquiring a selection instruction of a user, and editing the number of the scoring items, the weighting coefficients corresponding to the scoring items and/or the corresponding relationships between the scoring items and the corresponding step scoring coefficients according to the selection instruction.

10. A sleep evaluation display device, wherein the device comprises:
a signal acquisition module configured to acquire a sleep monitoring signal;
a type-by-type analysis module configured to analyze the sleep monitoring signal in sequence based on the plurality of scoring items according to a signal parsing strategy corresponding to each of the scoring items, to obtain signal parsing results corresponding to a plurality of scoring items respectively;
a searching module configured to search for a corresponding relationship between each of the scoring items and a corresponding step scoring coefficient according to the signal parsing results corresponding to the plurality of scoring items respectively, and determine a step scoring coefficient corresponding to each of the scoring items;
a calculation module configured to summarize the step scoring coefficients of all the scoring items to obtain an overall evaluation result of the sleep monitoring signal; and
a prompting module configured to prompt the overall evaluation result and the related step scoring coefficients.

11. The device according to claim 10, wherein the scoring items comprise: at least two of bradycardia, tachycardia, an actual sleep time point, a deep sleep proportion, a waking frequency, waking time, off-bed time, tachypnea, bradypnea, an apnea frequency, an off-bed frequency, a duration for falling asleep, a body movement and turning-over frequency, a breathing rate, a sleep duration, a heart rate, arrhythmia, a heartbeat pause frequency, a rapid-eye-movement (REM) sleep proportion, a non-rapid-eye-movement (NREM) sleep proportion and blood oxygen saturation.

12. The device according to claim 10, wherein the corresponding relationships between each scoring item and the corresponding step scoring coefficients are pre-stored to construct data corresponding relationships, wherein each scoring item is divided into different stages according to reference ranges, and each stage corresponds to a step scoring coefficient.

13. The device according to claim 10, wherein the step scoring coefficient corresponding to each scoring item is a numerical value or a percentage; and
the calculation module is configured to summarize the step scoring coefficients corresponding to all the scoring items to obtain the overall evaluation result of the sleep monitoring signal, or,
subtract the step scoring coefficients corresponding to all the scoring items from a basic score to obtain the overall evaluation result of the sleep monitoring signal, or,
perform weighted averaging on the step scoring coefficients corresponding to all the scoring items to obtain the overall evaluation result of the sleep monitoring signal.

14. The device according to claim 10, wherein the prompting module comprises at least one of the following units:
a first prompting unit configured to display or broadcast the overall evaluation result and the step scoring coefficient corresponding to each of the scoring items for summarization;
a second prompting unit configured to display or broadcast the overall evaluation result and the step scoring coefficients corresponding to all the scoring items;
a third prompting unit configured to display or broadcast the overall evaluation result, the corresponding relationship between each of the scoring items for summarization and the corresponding step scoring coefficient, and the corresponding step scoring coefficients; and
a fourth prompting unit configured to display or broadcast the overall evaluation result, the corresponding relationships between all the scoring items and the corresponding step scoring coefficients, and the corresponding step scoring coefficients.

15. The device according to claim 10, wherein according to the scoring items, the sleep monitoring signal comprises at least one signal or a combination of two or more signals selected from a body movement signal, a sleep heart rate signal, a sleep breathing rate signal, a breath noise signal, an electroencephalogram signal, an electrooculogram signal, an electromyogram signal and blood oxygen saturation detection data.

16. The device according to claim 10, wherein the type-by-type analysis module comprises at least one of the following units:
a body movement analysis unit configured to obtain signal parsing results corresponding to a plurality of scoring items related to body movement information according to the body movement signal, or according to the body movement signal in conjunction with the sleep heart rate signal and/or the sleep breathing rate signal;
a heart rate analysis unit configured to obtain signal parsing results corresponding to a plurality of scoring items related to a sleep heart rate according to the sleep heart rate signal;
a breathing rate analysis unit configured to obtain signal parsing results corresponding to a plurality of scoring items related to a sleep breathing rate according to the sleep breathing rate signal; and
a sleep state analysis unit configured to obtain a signal parsing result corresponding to a REM sleep proportion and/or an NREM sleep proportion according to at least one of the electroencephalogram signal, the electrooculogram signal and the electromyogram signal, and/or perform integral analysis on a combination of at least one of the electroencephalogram signal, the electrooculogram signal and the electromyogram signal, and at least one signal of the body movement signal, the sleep heart rate signal and the sleep breathing rate signal, to obtain signal parsing results corresponding to a plurality of scoring items related to sleep time .

17. A sleep evaluation equipment, comprising:
a storage module configured to store a signal parsing strategy corresponding to each of the scoring items and a corresponding relationship between each of the scoring items and a corresponding step scoring coefficient;
an interface module configured to acquire a sleep monitoring signal;
a processor configured to analyze the sleep monitoring signal in sequence based on the plurality of scoring items according to the signal parsing strategy corresponding to each of the scoring items to obtain signal parsing results corresponding to a plurality of scoring items respectively, search for a corresponding relationship between each of the scoring items and a corresponding step scoring coefficient according to the signal parsing results corresponding to the plurality of scoring items respectively, determine a step scoring coefficient corresponding to each of the scoring items, and summarize step scoring coefficients of all the scoring items to obtain an overall evaluation result of the sleep monitoring signal; and
a display and/or audio output module configured to display the overall evaluation result and the step scoring coefficients related thereto through the display, and/or broadcast the overall evaluation result and the step scoring coefficients related thereto through the audio output module.

18. The equipment according to claim 17, wherein the scoring items comprise at least two of: bradycardia, tachycardia, an actual sleep time point, a deep sleep proportion, a waking frequency, waking time, off-bed time, tachypnea, bradypnea, an apnea frequency, an off-bed frequency, a duration for falling asleep, a body movement and turning-over frequency, a breathing rate, a sleep duration, a heart rate, arrhythmia, a heartbeat pause frequency, a rapid-eye-movement (REM) sleep proportion, a non-rapid-eye-movement (NREM) sleep proportion and blood oxygen saturation.

19. The equipment according to claim 17, wherein the corresponding relationship between each of the scoring items and the corresponding step scoring coefficient is pre-stored to construct data corresponding relationships, wherein each of the scoring item is divided into different stages according to reference ranges, and each stage corresponds to a step scoring coefficient.

20. The equipment according to claim 17, wherein the step scoring coefficient corresponding to each scoring item is a numerical value or a percentage; and the processor is configured to summarize the step scoring coefficients corresponding to all the scoring items to obtain the overall evaluation result of the sleep monitoring signal; or, subtract the step scoring coefficients corresponding to all the scoring items from a basic score to obtain the overall evaluation result of the sleep monitoring signal; or, perform weighted averaging on the step scoring coefficients corresponding to all the scoring items to obtain the overall evaluation result of the sleep monitoring signal.

21. The equipment according to claim 17, wherein the display is configured to:
display the overall evaluation result and the step scoring coefficient corresponding to each of the scoring items for summarization;
display the overall evaluation result and the step scoring coefficients corresponding to all the scoring items;
display the overall evaluation result, the corresponding relationship between each of the scoring items for summarization and the corresponding step scoring coefficients, and the corresponding step scoring coefficients; or,
display the overall evaluation result, the corresponding relationships between all the scoring items and the corresponding step scoring coefficients, and the corresponding step scoring coefficients.

22. The equipment according to claim 17, wherein according to the scoring items, the sleep monitoring signal comprises at least one signal or a combination of two or more signals selected from a body movement signal, a sleep heart rate signal, a sleep breathing rate signal, a breath noise signal, an electroencephalogram signal, an electrooculogram signal, an electromyogram signal and blood oxygen saturation detection data.

23. The equipment according to claim 17, wherein the processor is further configured to execute at least one of the following steps:
obtaining signal parsing results corresponding to a plurality of scoring items related to a sleep breathing rate according to the sleep breathing rate signal;
obtaining signal parsing results corresponding to a plurality of scoring items related to a sleep heart rate according to the sleep heart rate signal;
obtaining signal parsing results corresponding to a plurality of scoring items related to body movement information according to the body movement signal, or according to the body movement signal in conjunction with the sleep heart rate signal and/or the sleep breathing rate signal;
obtaining a signal parsing result corresponding to a REM sleep proportion and/or an NREM sleep proportion according to at least one of the electroencephalogram signal, the electrooculogram signal and the electromyogram signal; and
performing integral analysis on a combination of at least one of the electroencephalogram signal, the electrooculogram signal and the electromyogram signal, and at least one signal of the body movement signal, the sleep heart rate signal and the sleep breathing rate signal, to obtain signal parsing results corresponding to a plurality of scoring items related to sleep time.

24. The equipment according to claim 17, wherein the display is utilized to display the scoring items, weighting coefficients corresponding the scoring items, and/or the corresponding relationships between the scoring items and the corresponding step scoring coefficients, and the equipment further comprises:
a human-computer interaction module configured to receive a selection instruction input by a user, and edit and save the number of the scoring items, the weighting coefficients corresponding to the scoring items and/or the corresponding relationships between the scoring items and the corresponding step scoring coefficients according to the selection instruction.
